# EUROPÄISCHE PATENTSCHRIFT

(11) **EP 0 513 577 B1**
(45) Veröffentlichungstag und Bekanntmachung des Hinweises auf die Patenterteilung: **18.01.1995**
(21) Anmeldenummer: 92107009.0
(22) Anmeldetag: 24.04.1992
(51) Int. Cl.: C25B 3/02, C07D 311/76

(54) **Verfahren zur Herstellung von 1-Alkoxyisochromanen**
Method for the preparation of 1-alkoxyisochromans
Procédé pour la préparation de 1-alkoxyisochromanes

(30) Priorität: 17.05.1991 DE 4116147
(43) Veröffentlichungstag der Anmeldung: 19.11.1992
(73) Patentinhaber: BASF Aktiengesellschaft, 67063 Ludwigshafen (DE)
(72) Erfinder: Hermeling, Dieter, Dr., W-6710 Frankenthal (DE)

(56) Entgegenhaltungen:
- EP-A- 0 011 712
- DE-A- 3 438 568

## Beschreibung

Die vorliegende Erfindung betrifft ein neues Verfahren zu Herstellung von 1-Alkoxyisochromanen der allgemeinen Formel I 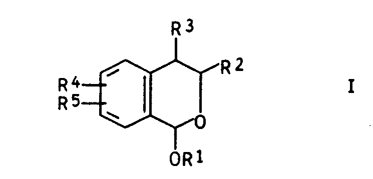
in der
- R¹: für einen Alkylrest steht,
- R² und R³: Wasserstoff oder Alkyl-, Cycloalkyl-, Aralkyl- oder Arylreste bezeichnen und
- R⁴ und R⁵: einen der Reste R² oder R³ oder Alkoxy-, Aryloxy- oder Halogenreste bedeuten.

Die Verbindungen I sind bekannt und dienen als Zwischenprodukte für die Synthese von Antitumorpharmazeutika, Antiallergica und Fungiziden.

Aus der DE-A 34 38 568 ist die Herstellung von 1-Alkoxyisochromanen durch Elektrooxidation von Isochromanen in Gegenwart von Alkanolen bekannt.

Dieses an sich vorteilhafte Syntheseverfahren hat jedoch den Nachteil, daß die Isochromane schlecht zugänglich sind.

Der Erfindung lag deshalb die Aufgabe zugrunde, die 1-Alkoxyisochromane I auf einfachere und wirtschaftlichere Weise zugänglich zu machen.

Demgemäp wurde ein neues Verfahren zur Herstellung von 1-Alkoxyisochromanen der allgemeinen Formel I 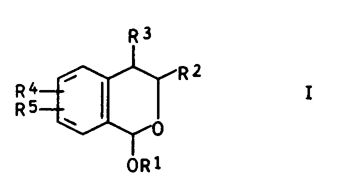
in der
- R¹: für einen Alkylrest steht,
- R² und R³: Wasserstoff oder Alkyl-, Cycloalkyl-, Aralkyl- oder Arylreste bezeichnen und
- R⁴ und R⁵: einen der Reste R² oder R³ oder Alkoxy-, Aryloxy- oder Halogenreste bedeuten,

gefunden, welches dadurch gekennzeichnet ist, daß man ein Gemisch aus einem o-Tolylethanol der allgemeinen Formel II 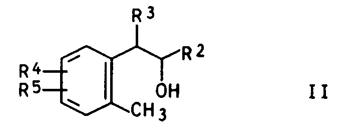
und einem Alkanol der allgemeinen Formel III

R¹-OH III

einer elektrochemischen Oxidation unterwirft.

Das erfindungsgemäße Verfahren läßt sich wie folgt veranschaulichen: 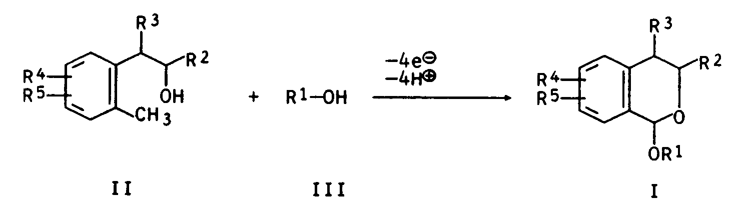
Die Ausgangsverbindungen II sind bekannt oder nach bekannten Methoden erhältlich, z.B. durch Umsetzung von Aromaten mit Epoxiden in Gegenwart von Lewis-Säuren (Bull. Chem. Soc. Jpn., 40, 2980 (1967)).

Unter den definitionsgemäßen Verbindungen II werden im Hinblick auf die Synthesen von Wirkstoffen solche bevorzugt, in denen die Reste folgende Bedeutung haben:
- Wasserstoff;
- C₁-C₂₀-Alkyl, vorzugsweise C₁-C₁₂-Alkyl, darunter vorzugsweise C₁-C₈-Alkyl wie n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, n-Hexyl, n-Heptyl, n-Octyl und vor allem Methyl und Ethyl;
- C₃-C₈-Cycloalkyl, vorzugsweise C₅-C₇-Cycloalkyl wie Cyclopentyl, Cycloheptyl und besonders Cyclohexyl;
- C₇-C₁₂-Aralkyl, vorzugsweise Phenylethyl und ganz besonders Benzyl;
- Aryl wie 1-Naphthyl, 2-Naphthyl und vorzugsweise Phenyl;
- C₁-C₂₀-Alkoxy, vorzugsweise C₁-C₁₂-Alkoxy, darunter vorzugsweise C₁-C₆-Alkoxy und ganz besonders Methoxy und Ethoxy;
- Aryloxy wie 1-Naphthyloxy, 2-Naphthyloxy und vor allem Phenoxy;
- Halogen wie vorzugsweise Brom und ganz besonders Fluor und Chlor.

Hierbei können die Alkyl-, Cycloalkyl- und Arylgruppen ihrerseits Substituenten tragen, vorzugsweise C₁-C₂-Alkylgruppen, C₁-C₂-Alkoxygruppen und Halogen.

Bevorzugte Ausgangsstoffe II sind:
- 2-(2-Hydroxyethyl)-1-methylbenzol
- 2-(2-Hydroxyethyl)-1,4-dimethylbenzol
- 4-tert.-Amyl-2-(2-hydroxyethyl)-1-methylbenzol
- 4-Ethoxy-2-(2-hydroxyethyl)-1-methylbenzol
- 4-tert.-Butoxy-2-(2-hydroxyethyl)-1-methylbenzol.

Im Hinblick auf die gewünschten Verfahrensprodukte I sind das 2-(2-Hydroxyethyl)-4-methoxy-1-methylbenzol und das 4-tert.-Butyl-2-(2-hydroxyethyl)-1-methylbenzol besonders bevorzugt.

Bevorzugte Verbindungen III sind C₁-C₆-Alkanole, darunter besonders C₁-C₄-Alkanole wie vor allem Methanol und Ethanol.

Um eine für die Elektrolyse ausreichende Leitfähigkeit des Elektrolyten zu gewährleisten, wird der Elektrolysemischung ein Hilfselektrolyt zugefügt. Als Hilfselektrolyte eignen sich beispielsweise Basen wie Alkalialkoholate und Alkalihydroxide. Besonders bevorzugt sind neutrale Hilfselektrolyte wie Fluoride, Tetrafluoroborate, Sulfonate, Sulfate, Phosphate und Phosphonate von Alkalimetallen sowie saure Hilfselektrolyte wie Schwefelsäure, Alkyl- und Arylsulfonsäuren.

Die Zusammensetzung des Elektrolyten kann in weiten Grenzen variiert werden. Diese enthalten bevorzugt
1 bis 49, vorzugsweise 5 bis 30 Gew.-% o-Tolylethanol II,
50 bis 98,9, vorzugsweise 70 bis 95 Gew.-% des Alkanols III R¹OH und
0,1 bis 5, vorzugsweise 0,2 bis 3 Gew.-% des Hilfselektrolyten.

Vorzugsweise nimmt man die elektrochemische Oxidation bei Stromdichten von 0,5 bis 25 A/dm², besonders bevorzugt bei 1 bis 10 A/dm² und bei Temperaturen von 0 bis 100°C, insbesondere bei 20 bis 80°C vor. Die Reaktion kann bei vermindertem Druck oder Normaldruck durchgeführt werden. Im Falle leichtsiedender Lösungsmittel nimmt man die Umsetzung vorzugsweise bei erhöhtem Druck bis 10 bar, insbesondere bis 3 bar vor. Die Ladungsmengen betragen in der Regel 2 bis 15 F/mol II, bevorzugt 2 bis 10 F/mol II.

Man kann die elektrochemische Oxidation sowohl in geteilter als auch in ungeteilter Zelle vornehmen. Bevorzugt wird eine ungeteilte Durchflußzelle verwendet, die zweckmäßigerweise zur Minimierung der Zellspannung geringe Elektrodenabstände von beispielsweise 0,25 bis 2 mm ermöglicht.

Die Elektrolysen können in an sich bekannter Weise sowohl kontinuierlich als auch diskontinuierlich durchgeführt werden.

Als Anodenmaterialien kommen im allgemeinen Edelmetalle wie Platin oder Oxide wie Ruthenium- und Chromoxid oder RuOₓTiOₓ-Mischoxide, bevorzugt jedoch Graphit in Betracht.

Als Kathodenmaterialien eignen sich in der Regel Stahl, Eisen, Kupfer, Nickel, Zink und Kohle sowie Edelmetalle wie Platin. Bevorzugtes Kathodenmaterial ist Graphit.

Eine wirtschaftlich sehr vorteilhafte Ausführungsform des erfindungsgemäßen Verfahrens besteht in der Durchführung der elektrochemischen Oxidation in ungeteilten Durchflußzellen unter Verwendung von Graphitelektroden.

Die Aufarbeitung erfolgt in an sich bekannter Weise, und zwar bevorzugt destillativ. Hierbei verfährt man zweckmäßigerweise so, daß man das überschüssige Alkanol abdestilliert, die ausgefallenen Salze abfiltriert und das Produkt destillativ gewinnt. Nicht umgesetztes Alkanol kann ohne weitere Reinigung zur Elektrolyse rückgeführt werden.

Die nach dem erfindungsgemäßen Verfahren auf einfache und wirtschaftliche Weise erhältichen 1-Alkoxyisochromane I sind wichtige Zwischenprodukte, z.B. für die Herstellung von Isochromanyltropolonen, die Antitumoraktivitäten aufweisen (Chem. Pharm. Bull., 31, 2952 (1983)) und von 4-Benzyl-1-(1-isochromanyl)-piperidin, einer Verbindung, die die Freisetzung von Histamin inhibitiert und damit als Antiallergicum wirkt. Einige 1-Alkoxyisochromane zeigen fungizide Eigenschaften (DE-A 34 38 568).

### Beispiele

### Beispiel 1

### Elektrosynthese von 1-Methoxy-6-methylisochroman

2-(2-Hydroxyethyl)-1,4-dimethylbenzol wurde in der wie folgt beschriebenen Elektrolysezelle unter den angegebenen Bedingungen oxidiert.
- Apparatur:: ungeteilte Zelle mit 11 bipolaren Elektroden
- Anode:: Graphit
- Elektrolyt:: 72,5 g (483 mmol) 2-(2-Hydroxyethyl)-1,4-dimethylbenzol, 2,9 g Natriumbenzolsulfonat, 650,0 g Methanol
- Kathode:: Graphit
- Stromdichte:: 3 A/dm²
- Elektrolysetemperatur:: 40°C

Die Elektrolyse wurde mit 5 F/mol 2-(2-Hydroxyethyl)-1,4-dimethylbenzol durchgeführt. Der Elektrolyt wurde während der Elektrolyse mit 190 l/h durch die Zelle geleitet. Nach Beendigung der Elektrolyse wurde das Methanol bei Normaldruck abdestilliert, und das ausgefallene Leitsalz wurde abfiltriert. Nach Reindestillation im Vakuum erhielt man neben 2,3 g nicht umgesetztem 2-(2-Hydroxyethyl)-1,4-dimethylbenzol 37 % 1-Methoxy-6-methylisochroman; Sdp. 82-84°C/1 mbar.

### Beispiel 2

### Elektrosynthese von 1-Methoxyisochroman

2-(2-Hydroxyethyl)-1-methylbenzol wurde in der wie folgt beschriebenen Elektrolysezelle unter den angegebenen Bedingungen oxidiert.
- Apparatur:: ungeteilte Zelle mit 9 bipolaren Elektroden
- Anode:: Graphit
- Elektrolyt:: 93,8 g (689 mmol) 2-(2-Hydroxyethyl)-1-methylbenzol,
6,2 g Natriumbenzolsulfonat,
525,0 g Methanol
- Kathode:: Graphit
- Stromdichte:: 6,4 A/dm²
- Elektrolysetemperatur:: 60°C

Die Elektrolyse wurde mit 8 F/mol 2-(2-Hydroxyethyl)-1-methylbenzol durchgeführt. Der Elektrolyt wurde während der Elektrolyse mit 20 l/h durch die Zelle geleitet. Die Aufarbeitung des Elektrolyseaustrags erfolgte wir in Beispiel 1. Durch Reindestillation im Vakuum wurden 36 % 1-Methoxyisochroman isoliert; Sdp. 65°C/1 mbar.

## Patentansprüche

1. Verfahren zur Herstellung von 1-Alkoxyisochromanen der allgemeinen Formel I 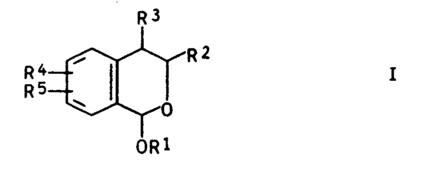 in der
R¹ für einen Alkylrest steht,
R² und R³ Wasserstoff oder Alkyl-, Cycloalkyl-, Aralkyl- oder Arylreste bezeichnen und
R⁴ und R⁵ einen der Reste R² oder R³ oder Alkoxy-, Aryloxy- oder Halogenreste bedeuten,
dadurch gekennzeichnet, daß man ein Gemisch aus einem o-Tolylethanol der allgemeinen Formel II 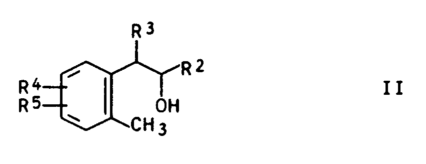 und einem Alkanol der allgemeinen Formel III
R¹-OH III
einer elektrochemischen Oxidation unterwirft.

2. Verfahren nach Anspruch 1, dadurch gekennzeichnet, daß man die elektrochemische Oxidation an Graphitelektroden durchführt.

3. Verfahren nach Anspruch 1 oder 2, dadurch gekennzeichnet, daß man die elektrochemische Oxidation in ungeteilten Durchflußzellen durchführt.

4. Verfahren nach den Ansprüchen 1 bis 3, dadurch gekennzeichnet, daß man als Alkanol III Methanol oder Ethanol verwendet.

## Claims

1. A process for preparing 1-alkoxyisochromans of the general formula I 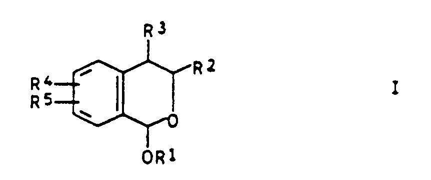 where
R¹ is alkyl,
R² and R³ are each hydrogen, alkyl, cycloalkyl, aralkyl or aryl, and
R⁴ and R⁵ each have the meaning of R² or R³ or are alkoxy, aryloxy or halo,
which comprises subjecting a mixture of an o-tolylethanol of the general formula II 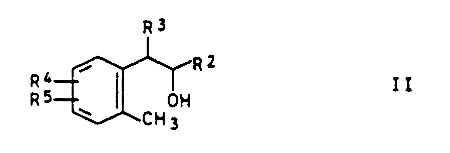 and an alkanol of the general formula III
R¹-OH III
to an electrochemical oxidation.

2. A process as claimed in claim 1, wherein the electrochemical oxidation is carried out on graphite electrodes.

3. A process as claimed in claim 1 or 2, wherein the electrochemical oxidation is carried out in undivided continuous flow cells.

4. A process as claimed in any of claims 1 to 3, wherein methanol or ethanol is used as alkanol III.

## Revendications

1. Procédé de préparation de 1-alcoxyisochromanes de formule générale I 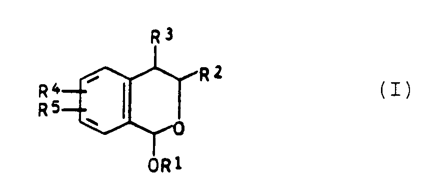 dans laquelle
R¹ est un reste alkyle,
R² et R³ définissent de l'hydrogène ou des restes alkyle, cycloalkyle, aralkyle ou aryle
et
R⁴ et R⁵ désignent un des restes R² ou R³ ou des restes alcoxy, aryloxy ou halogène,
caractérisé en ce que l'on soumet un mélange composé d'un o-tolyléthanol de formule générale II 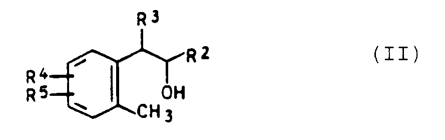 et d'un alcanol de formule générale III
R¹-OH (III)
à une oxydation électrochimique.

2. Procédé selon la revendication 1, caractérisé en ce que l'oxydation électrochimique est menée sur des électrodes au graphite.

3. Procédé selon la revendication 1 ou 2, caractérisé en ce que l'oxydation électrochimique est menée dans des cellules d'électrolyse non divisées.

4. Procédé selon les revendications 1 à 3, caractérisé en ce que l'on utilise, pour l'alcanol III, du méthanol ou de l'éthanol.
